Europäisches Patentamt

European Patent Office

Office européen des brevets

(19)

(11) Publication number: **0 119 859**
A2

# EUROPEAN PATENT APPLICATION

(12)

(21) Application number: 84301848.2

(22) Date of filing: 19.03.84

(51) Int. Cl.³: **C 12 P 21/00**, C 12 N 15/00, C 07 C 103/52, B 01 D 15/08, G 01 N 33/54, C 12 N 5/00

(30) Priority: 18.03.83 JP 45668/83

(43) Date of publication of application: 26.09.84
Bulletin 84/39

(84) Designated Contracting States: DE FR GB IT

(71) Applicant: KYOWA HAKKO KOGYO KABUSHIKI KAISHA, 6-1, Ohte-machi 1-chome, Chiyoda-ku Tokyo-to (JP)

(72) Inventor: Yoshida, Hajime, 9-18, Isobe, Sagamihara-shi Kanagawa-ken (JP)
Inventor: Fujiyoshi, Nobuo, 1-12-2, Asahi-machi, Machida-shi Toyko-to (JP)

(74) Representative: Watkins, Arnold Jack et al, European Patent Attorney Frank B. Dehn & Co. Imperial House 15-19 Kingsway, London WC2B 6UZ (GB)

(54) Monoclonal antibodies, processes for their preparation and methods for their use.

(57) Monoclonal antibodies specific for unglycosylated human interferon-$\beta$ are prepared by immunizing a mammal with unglycosylated human interferon-$\beta$ to produce cells capable of producing antibodies in the body of said mammal, fusing said cell with lined myeloma cells originating from a mammal other than the immunized mammal, selecting the resultant fused cells for the production of antibodies specific for unglycosylated human interferon-$\beta$, subjecting the selected fused cells to cloning to produce monoclones, selecting the resultant monoclones for reactivity with at least one member selected from unglycosylated human interferon-$\beta$ and other human interferons, culturing the selected monoclones, and recovering the desired monoclonal antibody from the culture thereby obtained.

Such monoclonal antibodies specific for unglycosylated human interferon-$\beta$ are used in processes for the purification and quantitative determination of unglycosylated human interferon-$\beta$.

- 1 -

"Monoclonal antibodies, processes for their preparation
and methods for their use"

The present invention relates to monoclonal antibodies specific for unglycosylated human interferon-β, processes for their preparation and methods for their use.

Human interferon-β is useful for treating and curing virally caused diseases and tumours. As a result of recent developments in DNA recombination methods, it is now possible to obtain large amounts of human interferon-β, by the action of microorganisms such as, for example, Escherichia coli.

Interferon-β, which may be obtained by culturing human fibroblasts, is in the form of a polypeptide bonded to a sugar moiety, whilst interferon-β obtained by DNA recombination techniques using Escherichia coli is in the form of unglycosylated interferon-β without sugar-bonding.

There is now a need to provide a method for purifying unglycosylated human interferon-β as well as a method for its quantitative determination, because it is expected that unglycosylated human interferon-β will be produced and utilized on an industrial scale, as opposed to interferon-β which may be obtained from fibroblast cells.

The present invention is based upon the discovery that unglycosylated human interferon-β (hereinafter referred to as HIFN-β) may with advantage be purified and quantitatively determined by the use of monoclonal antibodies which we have prepared by fusing the spleen cells of a mammal with myeloma cells, said spleen cells being collected from a mammal immunized with HIFN-β.

It was previously known to prepare monoclonal antibodies by the use of hybridoma cells which may be obtained by fusing mouse myeloma cells with the spleen cells of mice which have been immunized with interferon-β originating from human fibroblast cells [Japanese Patent Application as laid open to public inspection as Kokai Koho 194,788/82 and The Biology of the Interferon System, 67-71, Elsevier/Holland Biomedical Press. 1981]. However, it is difficult to use known monoclonal antibodies which are prepared by using interferon containing sugar chains as antigens for the purification and quantitative determination of unglycosylated HIFN-β.

The present invention is thus directed to providing anti-unglycosylated human interferon-β monoclonal antibodies, processes for their preparation and methods for thier use.

According to one feature of the present invention there are thus provided monoclonal antibodies specific for unglycosylated human interferon-β.

The monoclonal antibodies are preferably of the IgG isotype and advantageously selected from the $IgG_{2b}$, $IgG_{2a}$, and $IgG_1$ subclasses. The monoclonal antibodies are conveniently in substantially pure form. The monoclonal antibodies are advantageously presented in the form of an antiserum.

According to a further feature of the present invention there are provided monoclones for the preparation of the monoclonal antibodies of the present invention. The monoclones are preferably prepared according to the process described hereinafter for the preparation of the monoclonal antibodies of the present invention or by such steps thereof as are necessary. Selection of the monoclones may for example be effected on the basis of their reactivity with for example unglycosylated human interferon-β . The present invention also relates to fused (hybridoma) cells for the preparation of the monoclonal antibodies of the present invention e.g. prepared and/or selected according to the process described hereinafter for the preparation of the monoclonal antibodies

- 3 -

of the present invention or by such steps thereof as are necessary.

According to a further feature of the present invention there is provided a process for preparing monoclonal antibodies of the present invention which comprises immunizing a mammal with unglycosylated human interferon-$\beta$ to produce cells capable of producing antibodies in the body of said mammal, fusing said cells with lined myeloma cells originating from a mammal other than the immunized mammal, selecting the resultant fused cells for the production of antibodies specific for unglycosylated human interferon-$\beta$, subjecting the selected fused cells to cloning to produce monoclones, selecting the resultant monoclones for reactivity with at least one member selected from unglycosylated human interferon-$\beta$ and other human interferons, culturing the selected monoclones, and recovering the desired monoclonal antibody from the cultures thereby obtained.

Thus the monoclonal antibodies of the present invention may be prepared for example as described hereinafter:-

(1)    Preparation of immunized animal cells:

Mammals e.g. animals such as, for example, mice, rats, guinea pigs, rabbits, goats, horses, cattle and the like are immunized with unglycosylated human interferon-$\beta$ to produce cells capable of producing corresponding antibodies in the body of the mammal.

For example, unglycosylated human interferon-$\beta$ prepared by the process disclosed in Japanese Patent Application as laid open to public inspection as Kokai Koho 77654/82 may be used for the purpose of the present invention. For immunization, the interferon-$\beta$ may be injected subcutaneously, intraperitoneally or intravenously    for example, to 8-10 weeks old mice conveniently at a dose of $5 \times 10^5$-$10^7$ IU/animal. for example, in association with

- 4 -

a suitable adjuvant such as, for example, complete Freund's adjuvant, aluminium hydroxide gel, pertussis vaccine and the like, the interferon-$\beta$ and adjuvant e.g. being in equal amount. Advantageously subsequent immunizations may for example follow 2-10 times advantageously with an interval of 1-2 weeks. 3-7 days after each immunization, blood may be collected from the orbital plexus vein . . to investigate the anti-unglycosylated human inteferon-$\beta$ antibody titre for example by enzymoimmunoassay, for example using the solid phase sandwich method e.g. in the following manner [see "Koso Meneki Sokutei-ho" published by Igaku Shoin, Japan (1878)]:-

Unglycosylated HIFN-$\beta$ is conveniently diluted to a concentration of $10^6$ IU per ml with 1% bovine serum albumin (BSA)/PBS [disodium phosphate 1.83 g; monopotassium phosphate 0.21 g; sodium chloride 7.65 g and distilled water one l: pH=7.2] solution. An antigen solution [100 $\mu$l/well] is put into each well of a 96 well EIA plate [commercial product of Flow Laboratories, U.S.A.] and allowed to stand at 4°C overnight in order to coat the bottom surface of each well with said antigen. Each well is washed with deionized water, and then as a primary antibody, a multiply diluted sample containing mouse serum, supernatant of a culture of hybridoma cells and crude monoclonal antibodies [100 $\mu$l per well] is poured into each well and left at 4°C overnight.

The plate is washed once with deionized water and 6 times with 2M NaCl. Then, as a secondary antibody, a combined product of anti-mouse immunoglobulin F(ab')$_2$ of rabbit with urease [commercial product of Commonwealth Serum Laboratories, Australia (CSL); diluted with PBS solution to X 100] is poured into each well in an amount of 100 $\mu$l per well. After allowing to stand at room temperature for 2 hours, the plate is washed with

- 5 -

deionized water. Then, an urease substrate solution [100 µl per well; commercial product of CSL] is poured into each well and allowed to stand at room temperature for 10-30 minutes. After this, the reaction is discontinued for example by adding 1% methylthiolate (20 µl per well).

The antibody titre may for example be obtained by colorimetric determination at 600 nm. Where the resultant antibody titre rises to, for example, more than $10^3$ times the corresponding titre of normal mouse serum with respect to unglycosylated HIFN-β (determined by the OD value at 600 nm), such a mouse may be regarded as a useful source of the immunized animal cells.

In order to prepare hybridoma cells, unglycosylated HIFB-β is preferably administered to the immunized mouse 3-4 days before the hybridization. After additional immunizations, for example, the spleen and/or lymph node are collected in conventional manner from the mouse to prepare the desired cells.

(2) Preparation of myeloma cells:

As the myeloma cells, suitable lined cells of mouse origin may conveniently be used, for example myeloma cells of 8-azaguanine-resistant mice (of BALB/C mouse origin) such as, for example, P3-X63-Ag 8-UI (P3-U1) [Current Topics in Microbiology and Immunology, 81, 1-7 (1978)], P3-NSI/1-Ag4.1 (NS-1) [European J. Immunology, 6, 511-519 (1976)], SP2/0-Ag14 (SF-2) [Nature, 276, 269-270 (1978)], P3-X63-Ag8.653 (653) [J. Immunology, 123, 1548-1550 (1979)], P3-X63-Ag8 (X63) [Nature, 256, 495-497 (1975)] and the like.

These cell lines may be subcultured by using, for example, a known 8-azaguanine medium. 3-4 days before hybridization, the myeloma cells are preferably subcultured, for example by using a normal medium so as to ensure that the number of cells is more than 2 X $10^7$ on the

date of hybridization,

(3)    Fusion:

For example, the spleen cells thus-obtained and the myeloma cells obtained by the above-mentioned step (2) are well washed e.g. with MEM medium [commercial product of Nissui Seiyaku K.K., Japan] or a PBS solution, and they are combined together, the ratio of the number of spleen cells to the number of myeloma cells preferably being 5-10:1. A mixed solution of polyethylene glycol (PEG 1000-4000) (1-4 g), MEM medium (1-4 ml) and dimethylsulfoxide (0.5-1.0 ml) is for example added to the mixed cells with stirring in an amount of $0.1 - 1.0$ ml/$10^8$ spleen cells to effect the fusion in convention manner. If desired, HVJ (Sendai virus) may also be used for fusion,

The cell suspension is poured into each well of a culture plate for incubating at 35-40°C for 10-30 hours in an incubator containing $CO_2$ (3-7%). To each well of the plate, a HAT medium is added for culturing for 10-14 days. During the culturing, HAT medium is replaced by a new HAT medium, for example 3 times, in conventional manner. The cells are then cultured for example for 3-4 days by using HT medium. After this, part of the supernatant of the culture is collected to investigate anti-HIFN-β activity.

The most preferred method for selection of the antibody titre is the enzymoimmoassay method and if desired the radioimmunoassay method may be used solely or in combination. If desired, it is also possible to investigate the reactivities with other human interferons similarly. The hybridoma cells present in the wells which are recognised as those having sufficient titre are subjected to cloning (e.g. 2-4 times) by the limiting dilution method to obtain cells having a stable antibody titre, which are recognized as the hybridoma cells capable of producing anti-unglycosylated HIFN-β monoclonal antibodies.

(4)    Preparation of monoclonal antibodies:

The hybridoma cells recognized as those capable of producing anti-unglycosylated HIFN-$\beta$ monoclonal antibodies (2-4 X $10^6$ cells/mouse) are for example administered to a pristane[2,6,10,14-tetramethyl pentadecane]-treated BALB/c mouse (female; 8-10 weeks old) for example by abdominal injection. 10-21 days after this, ascites tumour is induced by the hybridoma cells. The ascites collected from the mouse is preferably centrifuged to remove solid impurities, and the salting-out of the supernatant is conveniently effected twice e.g. with 50% and 40% ammonium sulfate respectively, followed by for example dialysis against a PBS solution (pH=7.2) for 1-2 days. The resultant residual fraction may be qualified as partially purified monoclonal antibodies. If desired, the partially purified monoclonal antibodies may be treated with a column packed, for example, with DEAE Sepharose, Protein A Sepharose and the like to collect the IgG fraction.

· The neutralizing activity of the antiviral activity of the monoclonal antibodies may be assayed as follows:-

Interferon (200-500 IU per ml) and monoclonal antibodies are incubated at 37°C for 30 minutes, and the remaining activity of interferon may be determined by an assay system using WISH-Vesicular Stomatitis Virus (VSV) [Appl. Microbiol., 21, 723-725 (1971)]. The isotypes and subclasses of the antibodies may be identified by Ouchterlony's method [ "Menekigaku-jikken-nyumon, Seibutsukagaku Jikken-ho", published by Gakkai Shuppan Centre, Japan, page 74 (1981)].

The determination of protein may be effected according to Folin's method and the optical density at 280 nm [1.4 ($OD_{280}$) ÷ immunoglobulin 1 mg/ml].

In this manner, it has been confirmed that all monoclonal antibodies designated as KM 1-9 (see Example 1 hereinafter) obtained respectively from hybridoma cells designated as KM-1, KM-2, KM-3, KM-4, KM-5, KM-6, KM-7, KM-8 and KM-9 are of the isotype IgG and that KM-1 to KM-3 belong to $IgG_{2b}$, KM-4 and KM-5 belong to $IgG_{2a}$ and KM-6 to KM-9 belong to the subclass $IgG_1$.

The antigenic specificities and interferon neutralizing activities of KM-1 to KM-9 are indicated in items (5) and (6) in Example 1 as hereinafter described.

According to a further feature of the present invention there is provided a process for isolating unglycosylated human interferon-$\beta$ from a material containing the same, which comprises adsorbing the desired interferon-$\beta$ by using a monoclonal antibody specific for said human interferon-$\beta$. The process thus provides a method for purifying unglycosylated HIFN-$\beta$ using the monoclonal antibodies of the present invention. The process is conveniently effected by affinity chromatography known per se. The monoclonal antibodies of the present invention are conveniently immobilized by the use of an appropriate carrier e.g. by reaction with a cyanogen bromide-activated exchange resin e.g. CNBr-Sepharose 4B.

The present invention also relates to a carrier for adsorbing unglycosylated human interferon -$\beta$ which comprises a monoclonal antibody of the present invention immobilized on a carrier therefor.

Thus for example unglycosylated HIFN-$\beta$ may be purified using the monoclonal antibodies of the present invention in the following manner:-

A monoclonal antibody of the present invention (10 ml) may for example be dissolved in a PBS solution (1-10 ml) and then for example subjected to reaction with a suitable carrier such as, for example, CNBr-activated Sepharose 4B (1 ml; commercial product of Pharmacia Fine Chemicals AB., Sweden) to obtain an immobilized monoclonal antibody which may then be packed in a column. A solution

containing unglycosylated HIFN-$\beta$ (not more than $5 \times 10^8$ IU) may then be passed through the column. In this manner, it is possible to adsorb 90-95% of unglycosylated HIFN-$\beta$ on the column. For example, where a glycine-HCl buffered solution is used for elution, about 50% of the adsorbed unglycosylated HIFN-$\beta$ may be extracted. A single passage through the column may result in about a 3000 fold concentration of activity.

According to a further feature of the present invention there is provided a process for the quantitative determination of unglycosylated human interferon-$\beta$, which comprises using a monoclonal antibody specific for said human interferon-$\beta$ as a marker for enzymoimmunoassay and/or radioimmunoassay. Enzymoimmunoassay and radioimmunoassay techniques are known per se, the sandwich method being a convenient enzymoimmunoassay technique for use with the present invention.

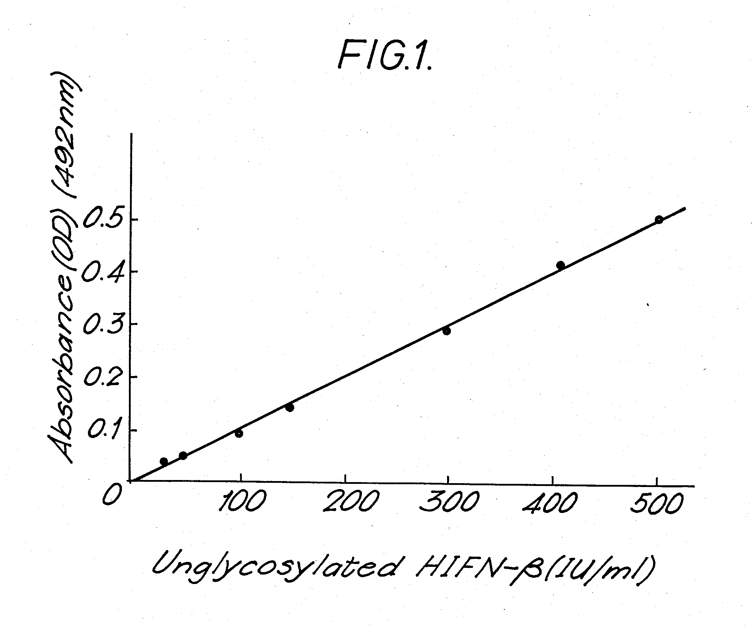

With regard to the drawing, Figure 1 shows the linear relationship between unglycosylated HIFN-$\beta$ content and absorbance (O.D.) at 492 mm.

- 10 -

Example 1

(1)    Preparation of the spleen cells of immunized mice:

10 female mice (BALB/c strain; 8 weeks old; purchased from Shizuoka-ken Jikken Dobutsu Nogyo Kyodo Kumiai, Japan) were immunized by abdominal injections of aluminium hydroxide gel (2 mg/mouse) and inactivated pertussis vaccine (1 X $10^9$ cells/mouse) as adjuvants and unglycosylated HIFN-$\beta$ (6.5 X $10^6$ IU/mouse) as antigen. Subsequent immunizations were effected at 2 week  intervals by abdominal injection of unglycosylated HIFN-$\beta$ (6.5 X $10^6$IU/mouse on each occasion). After the tertiary immunization, 5-7 days after each immunization, blood was collected from the orbital plexus vein -

to determine the anti-IFN-$\beta$ antibody titre in the serum by the above-mentioned enzymoimmunoassay method (solid phase sandwich method).

After the tertiary immunization, all mice showed positive antibody titres. However, in order to obtain monclonal antibodies of the IgG class, additional immunizations were effected 4 times. Thus, all mice were immunized 7 times in total. One week after the 7th immunization, unglycosylated HIFN-$\beta$ (6.5 X $10^6$ IU/mouse) was additionally administered to each mouse by abdominal injection. 3 days after this, the spleen cells were collected from the mice in conventional manner and used for the preparation of the fused cells.

(2)    Preparation of mouse myeloma cells:

P3-U1 (myeloma cells of 8-azaguanine-resistant mice were cultured by using a normal medium [RPMI-1640 containing glutamine (1.5 mM), 2-mercaptoethanol (5 X $10^{-5}$M),gentamicin (10 µg/ml) and foetal cattle serum (0.1ml/ml)] for 4 days to obtain more than 2 X $10^7$ cells.

(3)    Preparation of hybridoma cells:

The spleen cells of immunized mice (1 X $10^3$)were washed with MEM medium (commercial product of Nissui Seiyaku K.K., Japan) and mixed with 2 X $10^7$ P3-U1 myeloma

cells originating from 8-azaguanine-resistant mice. The mixture was centrifuged (1200 r.p.m./5 minutes) to give a precipitate of the mixed cells. The mixed cells were loosened and a solution (0.5 ml) containing a mixture of polyethylene glycol-1000 (PEG-1000; 2 g), MEM medium (2 ml) and dimethylsulfoxide (0.7 ml) was then added thereto. One minute after this, MEM medium ( 1 ml) was added to the cell suspension. Subsequently, MEM medium was added to the cell suspension five times (1 ml each time) with an interval of one minute between additions. After this, the total amount of MEM medium in the suspension was made up to 50 ml with further addition of the same medium. The suspension was centrifuged (900 r.p.m.)to remove the supernatant. The resultant mass of cells were loosened, a normal medium (100 ml) added thereto and the cells loosened by using a messpipet.

The cell suspension was poured into the wells of a 24 well culture plate (commercial product of Flow Laboratory, U.S.A.) in an amount of one ml per well, followed by incubating at 37°C for 24 hours by using a $CO_2$ incubator (5%). After adding hypoxanthine $(10^{-4}M)$, thimidine $(1.5 \times 10^{-5}M)$ and aminopterin $(4 \times 10^{-7}M)$, the culturing was effected at 37°C for 24 hours. After removal of the supernatant (1 ml), a new HAT medium ( 1 ml) was added to each medium for further culturing at 37°C for 10-14 days.

The presence of the fused cells grown in the form of colonies was found in certain wells, from which, on each occasion, the supernatant (1 ml) was removed and replaced by new HT medium (1 ml; prepared by removing aminopterin from HAT medium as above-mentioned) for culturing at 37°C. Such replacement by HT medium and further culturing was continued for the next 4 days. Then part of the supernatant was collected from each of the qualified cultures for assaying the titre of the anti-unglycosylated HIFN-$\beta$ antibody by the solid phase enzymoimmunoassay method as hereinbefore described.

The fused cells exhibiting sufficient antibody titres

0119859

- 12 -

were collected from the wells and subjected to cloning twice by the limiting dilution method to obtain clones having stability and qualified antibody titres. The resultant fused cells were recognized as the hybridoma cells capable of producing anti-unglycosylated HIFN-antibodies and designated as KM-1, KM-2, KM-3, KM-4, KM-5, KM-6, KM-7, KM-8 and KM-9.

(4) <u>Partial purification of monoclonal antibodies:</u>

Pristane [2,6,10,14-tetramethyl-pentadecane (0.5ml/ animal)] was abdominally administered to BALB/c female mice (8 weeks old) which were bred for 2 weeks. Then, the above-mentioned hybridoma cells (4 X $10^6$ cells/mouse on each occasion) were administered to the mice by abdominal injection. 10-21 days after this, an ascites tumour was induced by the hybridoma cells. 10-21 days after this, ascites (4-10 ml/each mouse) was collected from the mice, from which solid materials were removed by centrifugation. The salting-out of the supernatant is effected twice using 50 and 40% ammonium sulfate respectively, followed by dialysis against a PBS solution (pH=7.2) for 2 days to obtain partially purified monoclonal antibodies.

(5) Antigenic specificities of partially purified monoclonal antibodies:

The antigenic specificities of partially purified monoclonal antibodies were assayed by the solid phase enzymoimmunoassay method. The results shown in Table 1 were obtained by using as antigens: - unglycosylated HIFN-$\beta$ prepared by a DNA recombination technique; HIFN-$\alpha$ originating from Namalva; and bovine serum albumin (BSA).

TABLE 1

| Antibodies | Dilution | Binding activity $(OD_{600})$ | | |
|---|---|---|---|---|
| | | HIFN-$\beta$ | HIFN-$\alpha$ | BSA |
| Normal mouse serum | X $10^{-2}$ | 0.013 | 0.036 | 0.012 |
| Mouse serum (Immunized with HIFN-$\beta$ ) | X $10^{-2}$ X $10^{-2}$ | 0.426 | 0.043 | 0.001 |
| Partially purified monoclonal antibody | | | | |
| KM-1 | X $10^{-2}$ | 0.258 | 0.032 | 0.002 |
| KM-2 | X $10^{-2}$ | 0.243 | 0.028 | 0.005 |
| KM-3 | X $10^{-2}$ | 0.223 | 0.033 | 0.010 |
| KM-4 | X $10^{-2}$ | 0.214 | 0.035 | 0.015 |
| KM-5 | X $10^{-2}$ | 0.212 | 0.031 | 0.001 |
| KM-6 | X $10^{-2}$ | 0.298 | 0.029 | 0.008 |
| KM-7 | X $10^{-2}$ | 0.385 | 0.038 | 0.007 |
| KM-8 | X $10^{-2}$ | 0.376 | 0.039 | 0.006 |
| KM-9 | X $10^{-2}$ | 0.254 | 0.029 | 0.005 |

(6)     Neutralizing activity of partially purified monoclonal antibodies:

The above-mentioned method of assaying the neutralizing activities of the monoclonal antibodies for ungly-
cosylated HIFN-$\beta$ prepared by DNA recombination techniques,
and HIFN-$\alpha$ originating from Namalva was used.   The results
are shown in Table 2.

- 14 -

TABLE 2

| Antibodies | HIFN neutralizing activity % | |
| --- | --- | --- |
| | HIFN-$\beta$ | HIFN-$\alpha$ |
| Normal mouse serum | 0 | 0 |
| Mouse serum (immunized with HIFN-$\beta$ ) | 88 | 3 |
| Partially purified monoclonal antibodies | | |
| KM-1 | 76 | 5 |
| KM-2 | 77 | 8 |
| KM-3 | 88 | 6 |
| KM-4 | 47 | 8 |
| KM-5 | 82 | 5 |
| KM-6 | 61 | 5 |
| KM-7 | 61 | 9 |
| KM-8 | 2 | 2 |
| KM-9 | 55 | 9 |

It is apparent from Tables 1 and 2 that the monoclonal antibodies against unglycosylated HIFN-$\beta$ of the present invention specifically exhibit binding activity with and neutralizing activity for HIFN-$\beta$, and they thus are believed to be monoclonal antibodies specific for unglycosylated HIFN-$\beta$.

(7)    Classification of monoclonal antibodies:

Ouchterlony's method ["Menekigaku-jikken nyumon, Seibutsukagaku Jikken-ho" page 75 (1981) published by Gakkai Shuppan Centre, Japan] was used to identify the isotypes of the antibodies designated as KM-1 to KM-9.  It was found that all antibodies belong to the IgG class, KM-1 to KM-3 belonging to the $IgG_{2b}$, KM-4 and KM-5 belonging to the $IgG_{2a}$ and KM-6 to KM-9 belonging to the $IgG_1$ subclasses.

Example 2

Anti-unglycosylated HIFN-$\beta$ monoclonal antibodies

- 15 -

designated as KM-3 (10 mg/PBS; prepared by the method of Example 1) were subjected to reaction with CNBr-activated Sepharose 4B (1 ml; commercial product of Pharmacia Fine Chemicals AB., Sweden) to prepare an immobilized monoclonal antibody which was then packed in a column and extracted with a culture extract (5 ml; containing $2 \times 10^8$ IU of unglycosylated HIFN-$\beta$) to adsorb $1.87 \times 10^8$ IU (93.6 %) of said HIFN-$\beta$ onto the immobilized monoclonal antibody. After washing the column with a PBS solution, the immobilized antibody was applied with a glycine-HCl buffer solution (pH=2.3) to elute $9.4 \times 10^7$ IU (50 % of the adsorbed amount) of unglycosylated HIFN-$\beta$. The above-mentioned one stage treatment resulted in about a 3000 fold concentration of the activity. Where other monoclonal antibodies viz. KM-1, 2, 4, 5, 6, 7, 8 and 9 were used, unglycosylated HIFN-$\beta$ was purified in a similar manner to that described above.

Example 3

As a primary antibody, either one of KM-1, KM-2 and KM-3 was used at a concentration of 100 µg/ml. The primary antibody (100 µg/well) was poured into each well of a 96 well EIA plate (commercial product of Flow Laboratories, U.S.A.) and was allowed to stand at 4°C for 24 hours to coat the bottom surface of each well with the primary antibody. A 1 % solution of BSA (bovine serum albumin)/PBS (200 µl per well) was then applied to each well in order to coat the residual substances capable of binding protein on the bottom surface of each well with BSA and left at 4°C for 24 hours. The plate was washed well with deionized water, and 0.1-500 IU/ml of unglycosylated HIFN-$\beta$ was poured into each well in an amount of 100 µl/well, and allowed to stand at room temperature for 2 hours. Each well was well washed with deionized water. After this, one of the monoclonal antibodies selected from KM-4, KM-5, KM-6, KM-7, KM-8 and KM-9, all of which were combined with peroxidase of horseraddish origin (100 µg/ml), was poured into each well as a secondary antibody in an amount of 100 μg per well, and allowed to stand at room temperature for 2 hours. After washing each well with deionized water, a substrate solution (150 μl per well) containing o-phenylenediamine (40 mg) phosphoric acid/citric acid buffer solution (pH 5.1; 100 ml) and 30 % $H_2O_2$ (40 ml) was poured into each well and allowed to stand at room temperature for 30 minutes to carry out the enzymatic reaction which was then discontinued by adding $2MH_2SO_4$ to each well.

The antibody titres shown in Fig. 1 were assayed colorimetrically based upon optical absorbance at 492 nm, and this showed that the unglycosylated HIFN-β content is linearly proportional to the optical absorbance at 492 nm. (See Figure 1). It is thus possible to use the monoclonal antibodies of the present invention for the quantitative determination of unglycosylated HIFN-β by means of the solid phase sandwich method of enzymoimmunoassay.

It is also possible, if desired, to carry out the quantitative determination of unglycosylated HIFN-β by modifying the method as hereinbefore described, and the radioimmunoassay method may also be used instead of the enzymoimmunoassay method.

0119859

- 17 -

<u>Claims</u>

1.   Monoclonal antibodies specific for unglycosylated human interferon-$\beta$.

2.   Monoclonal antibodies as claimed in claim 1 of the IgG isotype.

3.   Monoclonal antibodies as claimed in claim 2 of the $IgG_{2b}$, $IgG_{2a}$ and/or $IgG_1$ subclass.

4.   Monoclonal antibodies as claimed in any one of claims 1 to 3 in the form of an antiserum.

5.   A process for preparing monoclonal antibodies as defined in claim 1, which  comprises immunizing a mammal with unglycosylated human interferon-$\beta$ to produce cells capable of producing antibodies in the body of said mammal, fusing said cells with lined myeloma cells originating from a mammal other than the immunized mammal, selecting the resultant fused cells for the production of antibodies specific for unglycosylated human interferon-$\beta$, subjecting the selected fused cells to cloning to produce monoclones, selecting the resultant monoclones for reactivity with at least one member selected from unglycosylated human interferon-$\beta$ and other human interferons, culturing the selected monoclones, and recovering the desired monoclonal antibody from the culture thereby obtained.

6.   A process as claimed in claim 5 wherein the mammal is a mouse.

7.  ·  A process as claimed in claim 5, in which the mammal is selected from rats,  guinea pigs, rabbits, goats, horses and cattle.

8.   A process for isolating unglycosylated human interferon-$\beta$ from a material containing the same, which comprises adsorbing the desired interferon-$\beta$ by using a monoclonal antibody specific for said human interferon-$\beta$ .

9.   A process as claimed in claim 8, in which the adsorption is effected by affinity chromatography.

10.    A column for adsorbing unglycosylated human interferon -$\beta$ which comprises a monoclonal antibody as defined in any one of claims 1 to 3 immobilized on a carrier therefor.

11.    A process for the quantitative determination of unglycosylated human interferon-$\beta$, which comprises using a monoclonal antibody specific for said human interferon-$\beta$ as a marker for enzymoimmunoassay and/or radioimmunoassay.

12.    Monoclones for the preparation of monoclonal antibodies as defined in any one of claims 1 to 3.

13.    Monoclones for the preparation of monoclonal antibodies as defined in any one of claims 1 to 3 prepared and selected by a process as defined in any one of claims 5 to 7.

14.    Hybridoma cells for the preparation of monoclonal antibodies as defined in any one of claims 1 to 3.

15.    Hybridoma cells for the preparation of monoclonal antibodies as defined in any one of claims 1 to 3 prepared and selected by a process as defined in any one of claims 5 to 7.

1/1

0119859

FIG.1.

Absorbance(OD) (492nm) vs Unglycosylated HIFN-β(IU/ml)